# EUROPÄISCHE PATENTANMELDUNG

(11) **EP 3 756 726 A2**
(43) Veröffentlichungstag der Anmeldung: **30.12.2020**
(21) Anmeldenummer: 20181459.7
(22) Anmeldetag: 22.06.2020
(51) Int. Cl.: A61N 1/362, A61N 1/372, A61N 1/375

(54) **SCHRITTMACHERNETZWERK**

(30) Priorität: 28.06.2019 DE 102019117586; 15.07.2019 DE 102019119117; 02.09.2019 DE 102019123464; 11.09.2019 DE 102019124435
(71) Anmelder: Mehnert, Walter, 85521 Ottobrunn (DE); Theil, Thomas, 82340 Feldafing (DE)
(72) Erfinder: Mehnert, Walter, 85521 Ottobrunn (DE); Theil, Thomas, 82340 Feldafing (DE)
(74) Vertreter: Kilian Kilian & Partner

(57) **Zusammenfassung**

Die Erfindung betrifft ein drahtloses Schrittmachernetzwerk zur Implantation in einen Körper eines Lebewesens und zur Steuerung einer Körperfunktion, wobei das Schrittmachernetzwerk aufweist:
eine elektronische Schrittmachereinheit (1), die als ein Master in dem Schrittmachernetzwerk agiert, beinhaltend
• einen Elektrodenabschnitt (2), der bestimmungsgemäß an einem ersten Körperabschnitt zu befestigen ist, und
• eine Elektronik; und

eine elektronische Schrittmachereinheit (1'), die als ein Slave in dem Schrittmachernetzwerk agiert, beinhaltend
• einen Elektrodenabschnitt (2'), der bestimmungsgemäß an einem zweiten Körperabschnitt zu befestigen ist, und
• eine mit dem Elektrodenabschnitt verbundene Elektronik, die eingerichtet ist, einen Impuls, insbesondere einen Spannungsimpuls, zu erzeugen und diesen über den Elektrodenabschnitt an den zweiten Körperabschnitt abzugeben; wobei

die als Master agierende Schrittmachereinheit (1) und die als Slave agierende Schrittmachereinheit (1') zur Steuerung der Körperfunktion drahtlos zusammenzuwirken.

## Beschreibung

Die Erfindung betrifft ein drahtloses Schrittmachernetzwerk zur Implantation in einen Körper eines Lebewesens und zur Steuerung einer Körperfunktion.

Schrittmacher sind im Stand der Technik allgemein bekannt und werden bei einer Vielzahl von verschiedenen medizinischen Indikationen eingesetzt.

Beispielsweise werden Schrittmacher bei Erkrankungen des menschlichen Herzen als sogenannte Zweikammer-Herzschrittmacher eingesetzt.

Die wesentlichen Komponenten eines solchen Zweikammer-Herzschrittmachers bilden an einem Gehäuse befestigte Kabelelektroden, die mit den verschiedenen Kammern des erkrankten Herzen des Patienten verbunden werden, und eine in dem Gehäuse aufgenommene Elektronik, die über die Elektroden Funktionen der Kammern überwachen und bei Feststellen von Fehlfunktionen an diese Stimulationsimpulse abgeben kann. Alle genannten Komponenten sind miteinander kabelgebunden und werden heutzutage vollständig in den menschlichen Körper implantiert.

In der Regel wird das Gehäuse in der Nähe des Herzen, beispielsweise unterhalb des Brustbeins oder Schlüsselbeins, angeordnet und die Kabelelektroden an die zu stimulierenden Herzkammern geführt. Die eingesetzten Kabelelektroden sind hierfür relativ lang ausgebildet, um die Herzkammern innerhalb des Körpers des Patienten erreichen zu können.

Einer gewünschten Miniaturisierung der bekannten Zweikammer-Herzschrittmacher sind bei einem solchen Aufbau Grenzen gesetzt.

Außerdem sind die Kabelelektroden Schwachstellen, weil notwendige Isolierungen zu Abstoßungsreaktionen des Körpers des Patienten führen können und aufgrund von insbesondere durch mechanische Reibung bzw. Verformung auftretenden Alterungserscheinungen einen Austausch der Kabelelektroden nach einer gewissen Zeitspanne erforderlich machen.

Vor obigem Hintergrund ist es Aufgabe der vorliegenden Erfindung, Möglichkeiten zu schaffen, verschiedene - in einem Abstand zueinander liegende - Körperabschnitte stimulieren und einen hierfür notwendigen elektronischen Aufbau verkleinern zu können.

Diese Aufgabe wird mit einem Schrittmachernetzwerk gemäß Patentanspruch 1 gelöst. Bevorzugte Ausführungsformen sind Gegenstand der abhängigen Patentansprüche.

Grundgedanke der Erfindung ist es, dass Schrittmacher, die verschiedene Körperabschnitte zu stimulieren haben, dadurch aufgebaut werden, dass einzelne den jeweiligen Körperabschnitten zugeordnete Schrittmachereinheiten zu einem Schrittmachernetzwerk miteinander drahtlos verbunden werden. Der Aufbau eines drahtlosen Schrittmachernetzwerkes ermöglicht, dass zur Stimulation notwendige Elektrodenabschnitte sehr kurz bzw. klein und eine entsprechend zugeordnete Elektronik kleiner ausgebildet werden können.

*Gemäß obigem Grundgedanken der Erfindung beinhaltet ein drahtloses Schrittmachernetzwerk zur Implantation in einen Körper eines Lebewesens und zur Steuerung einer Körperfunktion:*
*eine elektronische Schrittmachereinheit, die als ein Master in dem Schrittmachernetzwerk agiert, welche aufweist*
   - *einen Elektrodenabschnitt, der bestimmungsgemäß an einem ersten Körperabschnitt zu befestigen*/*anzuordnen ist, und*
   - *eine Elektronik, die eingerichtet ist, eine Funktion bzw. Eigenaktion des ersten Körperabschnittes, bevorzugt über den Elektrodenabschnitt oder eine gesonderte Überwachungselektrode, zu überwachen und*/*oder einen Impuls, insbesondere einen Spannungsimpuls, zu erzeugen und diesen über den Elektrodenabschnitt an den ersten Körperabschnitt abzugeben; und*
*eine elektronische Schrittmachereinheit, die als ein Slave in dem Schrittmachernetzwerk agiert, welche aufweist*
   - *einen Elektrodenabschnitt, der bestimmungsgemäß an einem zweiten Körperabschnitt zu befestigen*/*anzuordnen ist, und*
   - *eine mit dem Elektrodenabschnitt verbundene Elektronik, die eingerichtet ist, einen Impuls, insbesondere einen Spannungsimpuls, zu erzeugen und diesen über den Elektrodenabschnitt an den zweiten Körperabschnitt abzugeben; wobei*
*die als Master agierende Schrittmachereinheit und die als Slave agierende Schrittmachereinheit eingerichtet sind, zur Steuerung der Körperfunktion drahtlos zusammenzuwirken, indem die Elektronik der als Slave agierenden Schrittmachereinheit (i) Information über die Funktion bzw. Eigenaktion des ersten Körperabschnittes von der als Master agierenden Schrittmachereinheit und*/*oder über die Abgabe des Impulses an den ersten Körperabschnitt erhält, und (ii) basierend auf der Information entscheidet, ob und*/*oder wann die Abgabe des Impuls an den zweiten Körperabschnitt erfolgt.*

*Bevorzugt kann die Elektronik der als Slave agierenden Schrittmachereinheit* - *wie auch die der als Master agierenden Schrittmachereinheit - eingerichtet sein, eine Funktion bzw. Eigenaktion des zweiten Körperabschnittes, bevorzugt über den Elektrodenabschnitt oder eine gesonderte Überwachungselektrode, zu überwachen und*/*oder den Impuls, insbesondere den Spannungsimpuls, zu erzeugen und diesen über den Elektrodenabschnitt an den zweiten Körperabschnitt abzugeben.*

In einer bevorzugten Ausführungsform, auf die die Erfindung nicht eingeschränkt ist, übernimmt das erfindungsgemäße Schrittmachernetzwerk die Funktion eines Mehrkammer-Herzschrittmachers, beispielsweise eines Zweikammer-Herzschrittmachers.

D. h. die von dem erfindungsgemäßen Schrittmachernetzwerk zu übernehmende Steuerung der Körperfunktion betrifft beispielsweise die Steuerung des menschlichen Herzschlages, indem die einzelnen Herzkammern überwacht und/oder durch Impulse stimuliert werden.

Hierfür wird bestimmungsgemäß die als Master agierende Schrittmachereinheit beispielsweise einem Vorhof (Atrium) des menschlichen Herzen, der dem ersten Körperabschnitt entspricht, und die als Slave agierende Schrittmachereinheit einer Hauptkammer (Ventrikel) des menschlichen Herzen, die dem zweiten Körperabschnitt entspricht, zugeordnet.

Die Zuordnung erfolgt insbesondere dadurch, dass die Elektrodenabschnitte der als Master agierenden Schrittmachereinheit und der als Slave agierenden Schrittmachereinheit an dem jeweiligen ersten und zweiten Körperabschnitt verankert werden. Die Verankerung kann beispielsweise durch eine Drahtspirale, die bei Implantation in ein Gewebe des menschlichen Herzen gedreht wird, erfolgen. Alternativ können für die Verankerung Widerhaken, die sich bei Eindringen in das Gewebe verhaken, dienen. Weiterhin alternativ kann der Elektrodenabschnitt der als Master und der als Slave agierenden Schrittmachereinheit als flache Elektrode ausgebildet sein, die auf einer Außenoberfläche eines im Folgenden noch erläuterten Gehäuses/Hülle der jeweiligen Schrittmachereinheit freiliegt.

Wenn das erfindungsgemäße Schrittmachernetzwerk hierüber hinausgehend die Stimulation einer weiteren Herzkammer übernehmen soll, d. h. dass das erfindungsgemäße Schrittmachernetzwerk die Funktion eines Dreikammer-Herzschrittmachers übernimmt, ist bevorzugt eine weitere als Slave agierende Schrittmachereinheit in dem Schrittmachernetzwerk vorgesehen, die über einen entsprechenden Elektrodenabschnitt an einem dritten Körperabschnitt verankert ist.

Die genannten Schrittmachereinheiten interagieren zusammen kabellos, weshalb sie jeweils äußerst nah an den entsprechenden Körperabschnitten angeordnet und die Elektrodenabschnitte sehr klein und bevorzugt ohne eine entsprechende Kunststoffisolierung ausgebildet werden können.

*Bevorzugt ist das erfindungsgemäße Schrittmachernetzwerk so aufgebaut, dass die als Master agierende Schrittmachereinheit eine Sendeeinheit aufweist und eingerichtet ist, die Information über die Sendeeinheit zu versenden, und dass die als Slave agierende Schrittmachereinheit eine Empfangseinheit aufweist und eingerichtet ist, die Information dadurch zu erhalten, dass sie die von der Sendeeinheit versendete Information über die Empfangseinheit empfängt.*

Die Sendeeinheit kann auf verschiedenen Funktechniken basieren, beispielsweise Bluetooth, insbesondere Low Energy Bluetooth, oder allgemein auf Funktechniken mit sehr hohen oder sehr niedrigen Funkfrequenzen, wobei letztere bevorzugt sind.

*Weiterhin bevorzugt kann das erfindungsgemäße Schrittmachernetzwerk so ausgestaltet sein, dass die als Slave agierende Schrittmachereinheit eine Detektionseinheit aufweist und eingerichtet ist, die Information dadurch zu erhalten, dass sie über die Detektionseinheit den von der als Master agierenden Schrittmachereinheit abgegebenen Impuls detektiert.*

Die als Master agierende Schrittmachereinheit benötigt in diesem Fall insbesondere keine Sendeeinheit.

Wenn das erfindungsgemäße Schrittmachernetzwerk die Funktion eines Zweikammer-Herzschrittmachers übernimmt, kann mit dem im Vorhergehenden erläuterten Aufbau des erfindungsgemäßen Schrittmachernetzwerks beispielsweise folgende Funktionsweise durch das Schrittmachernetzwerk realisiert werden:
- Die Elektronik der als Master agierenden und dem Vorhof (Atrium) bzw. dem ersten Körperabschnitt zugeordneten Schrittmachereinheit überwacht die Funktion bzw. Eigenaktion des Vorhofs, bevorzugt über ihren Elektrodenabschnitt oder über die gesonderte Überwachungselektrode.
   Wenn die Elektronik im Rahmen der Überwachung des ersten Körperabschnitt eine entsprechende Eigenaktion feststellt, übermittelt sie diese Information, beispielsweise über die Sendeeinheit, an die als Slave agierende Schrittmachereinheit, die die entsprechende Information beispielsweise über ihre Empfangseinheit empfängt.
   Die als Slave agierende Schrittmachereinheit entscheidet basierend auf der empfangenen Information, wann sie über ihren entsprechenden Elektrodenabschnitt den Impuls an die Kammer (Ventrikel) bzw. an den zweiten Körperabschnitt abgibt. Dies erfolgt beispielsweise nach Erhalt der Information dadurch, dass die Elektronik der als Slave agierenden Schrittmachereinheit nach Ablauf einer bestimmten Zeitspanne (A/V-Intervall) unabhängig von einer Eigenaktion des zweiten Körperabschnitts immer den Impuls erzeugt und diesen zur Stimulation über ihren Elektrodenabschnitt an den zweiten Körperabschnitt abgibt.

Im Allgemeinen kann, wie bereits erläutert, die als Slave agierende Schrittmachereinheit bevorzugt ebenfalls eingerichtet sein, eine Funktion bzw. Eigenaktion des zweiten Körperabschnitts, bevorzugt über ihren Elektrodenabschnitt oder die weitere Überwachungselektrode, zu überwachen.

Durch diese bevorzugte Ausgestaltung der als Slave agierenden Schrittmachereinheit ergibt sich eine bevorzugte Abwandlung obiger Funktion dahingehend, dass die Elektronik der als Slave agierenden Schrittmachereinheit den Impuls nur dann erzeugt und über ihren Elektrodenabschnitt an den zweiten Körperabschnitt abgibt, wenn im Rahmen der Überwachung des zweiten Körperabschnittes bis zum Ablauf der genannten Zeitspanne (A/V-Intervall) keine Funktion bzw. Eigenaktion des zweiten Körperabschnitt durch die Elektronik festgestellt wurde. D.h. die Elektronik der als Slave agierenden Schrittmachereinheit entscheidet ob und, wenn ja, wann der Impuls abzugeben ist.

Im Rahmen der erläuterten Funktionsweise des erfindungsgemäßen Schrittmachernetzwerks ist die Elektronik der als Master agierenden Schrittmachereinheit eingerichtet, die Funktion bzw. Eigenaktion des ersten Körperabschnittes zu überwachen. Die erläuterte Überwachungsfunktion kann für bestimmte medizinische Indikationen ausreichend sein, beispielsweise für den Fall, dass die Funktion bzw. Eigenaktion des Vorhofs ungestört vorliegt und keiner Stimulation bedarf. Anders ausgedrückt kann die als Master agierende Schrittmachereinheit ausschließlich die Funktion der Überwachung des ersten Körperabschnittes übernehmen.

Der umgekehrte Fall, dass die Elektronik der als Master agierenden Schrittmachereinheit keine Überwachungsfunktion beinhaltet, sondern eingerichtet ist, den Impuls immer zu erzeugen und an den ersten Körperabschnitt abzugeben, ist ebenfalls als bevorzugte Variante denkbar. Beispielsweise lässt sich durch das erfindungsgemäße Schrittmachernetzwerk dann ein Zweikammer-Herzschrittmacher aufbauen, der folgende Funktionsweise realisiert:
- Die Elektronik der als Master agierenden und dem Vorhof (Atrium) bzw. dem ersten Körperabschnitt zugeordneten Schrittmachereinheit erzeugt immer den Impuls und gibt diesen über den entsprechenden Elektrodenabschnitt an den ersten Körperabschnitt ab.
   Die als Master agierende Schrittmachereinheit übermittelt diese Information, beispielsweise über die Sendeeinheit, an die als Slave agierende Schrittmachereinheit, die die entsprechende Information beispielsweise über ihre Empfangseinheit empfängt. Alternativ kann die als Slave agierende Schrittmachereinheit die Information auch über ihre Detektionseinheit bevorzugt erhalten; für diesen Fall benötigt die als Master agierende Schrittmachereinheit keine Sendeeinheit.
   Die als Slave agierende Schrittmachereinheit entscheidet basierend auf der empfangenen/erhaltenen Information über die Abgabe des Impuls an die Kammer (Ventrikel) bzw. an den zweiten Körperabschnitt. Dies kann, wie im Vorhergehenden erläutert, entweder unabhängig, d. h. ohne Überwachung der Eigenaktion des zweiten Körperabschnittes, oder abhängig, d. h. mit Überwachung der Eigenaktion des zweiten Körperabschnittes, erfolgen.

Letztendlich ist im Rahmen der Erfindung auch der kombinierte Fall, dass die Elektronik der als Master agierenden Schrittmachereinheit beide Funktionen aufweist, d. h. den ersten Körperabschnitt überwachen und bei Bedarf den Impuls erzeugen/abgeben kann, denkbar. Beispielsweise lässt sich durch das erfindungsgemäße Schrittmachernetzwerk dann ein Zweikammer-Herzschrittmacher aufbauen, der folgende Funktionsweise realisiert:
- Die Elektronik der als Master agierenden und dem Vorhof (Atrium) bzw. dem ersten Körperabschnitt zugeordneten Schrittmachereinheit überwacht die Funktion bzw. Eigenaktion des Vorhofs, bevorzugt über ihren Elektrodenabschnitt oder über eine gesonderte Überwachungselektrode. Wenn die Elektronik im Rahmen der Überwachung des ersten Körperabschnitts keine entsprechende Eigenaktion feststellt, erzeugt sie den entsprechenden Impuls und gibt diesen über den Elektrodenabschnitt an den ersten Körperabschnitt ab.
   Die Elektronik übermittelt anschließend entweder die Information, dass die Überwachung eine Eigenaktion bzw. Funktion des ersten Körperabschnittes festgestellt hat, oder die Information, dass der Impuls an den ersten Körperabschnitt abgegeben wurde, an die als Slave agierende Schrittmachereinheit, beispielsweise über die bereits erwähnte Sendeeinheit.
   Die als Slave agierende Schrittmachereinheit entscheidet basierend auf der empfangenen Information über die Abgabe des Impuls an die Kammer (Ventrikel) bzw. an den zweiten Körperabschnitt. Dies kann, wie im Vorhergehenden erläutert, entweder unabhängig, d. h. ohne Überwachung der Eigenaktion des zweiten Körperabschnittes, oder abhängig, d. h. mit Überwachung der Eigenaktion des zweiten Körperabschnittes, erfolgen.

*Die als Master agierende Schrittmachereinheit und*/*oder die als Slave agierende Schrittmachereinheit des dem Grundgedanken der Erfindung entsprechenden* Schrittmachernetzwerkes *beinhaltet bevorzugt:*
*einen Energiespeicher, z.B. einen Akkumulator oder einen Kondensator (beispielsweise ein Gold-Cap), zur Versorgung seiner Elektronik mit elektrischer Energie, der nach Entladung mit elektrischer Energie wieder aufgeladen werden kann.*

Die Art des Energiespeichers kann beliebig gewählt werden. Beispielsweise kann der Energiespeicher ein Akkumulator, vorzugsweise ein Lithium-Ionen-Akkumulator, sein. Alternativ kann der Energiespeicher ein Kondensator mit bevorzugt geringer Selbstentladung sein. Der Energiespeicher kann bevorzugt hermetisch eingekapselt sein, sodass von ihm keine Gefahr für den Körper des Lebewesens ausgeht.

*Ferner beinhalten die als Master agierende Schrittmachereinheit und*/*oder die als Slave agierende Schrittmachereinheit des dem Grundgedanken der Erfindung entsprechenden* Schrittmachernetzwerkes *bevorzugt jeweils:*
*einen mit dem Energiespeicher elektrisch verbundenen Ladeimpulserzeugungsabschnitt, der derart eingerichtet ist, dass er an den Energiespeicher einen Ladeimpuls zum Wiederaufladen des Energiespeichers abgeben kann, wobei der Ladeimpulserzeugungsabschnitt einen Magnetisierungsabschnitt mit ausgerichteten magnetischen Domänen beinhaltet, der durch ein sich veränderndes (externes) Magnetfeld derart kontaktlos beeinflussbar ist, dass in ihm ab Erreichen einer bestimmten Feldstärke (Amplitude) eine zur Erzeugung des Ladeimpulses führende über den Magnetisierungsabschnitt laufende Ummagnetisierungswelle, hervorgerufen durch die fortlaufend umgepolten magnetischen Domänen, auftritt.*

Das genannte externe bzw. extern erzeugte Magnetfeld wird für das kontaktlose Wiederaufladen des Energiespeichers bevorzugt durch das im Folgenden noch erläuterte erfindungsgemäße Ladegerät erzeugt.

Der Magnetisierungsabschnitt des Ladeimpulserzeugungsabschnittes weist gleich ausgerichtete magnetische Domänen auf, die gemeinsam durch das sich verändernde extern erzeugte Magnetfeld beeinflusst werden können. Wenn das extern erzeugte Magnetfeld in einem bestimmten Bereich des Magnetisierungsabschnittes eine bestimmte Amplitude bzw. Feldstärke, die in der Größenordnung weniger Millitesla (kleiner gleich 10mT) liegt, erreicht, magnetisieren sich die Domänen in diesem Bereich um (Umklappen der sog. Weiß'schen Bezirke), wodurch die genannte Ummagnetisierungswelle über den Magnetisierungsabschnitt zu laufen beginnt, so wie es z. B. bei einem im Folgenden noch erwähnten Wiegand- oder Impulsdraht der Fall ist.

Die Form des Magnetisierungsabschnittes ist beliebig.

Wenn die Stärke des extern erzeugten Magnetfeldes beispielsweise an einem Ende des Magnetisierungsabschnittes die bestimmte Amplitude bzw. Stärke erreicht, beginnt die Ummagnetisierungswelle an diesem Ende des Magnetisierungsabschnittes zu laufen, bis sie das andere Ende des Magnetisierungsabschnittes erreicht. Physikalisch gesehen handelt es sich bei der so auftretenden Ummagnetisierungswelle um im Wesentlichen eine Blochwand, die über den Magnetisierungsabschnitt läuft.

Die Ummagnetisierung des Magnetisierungsabschnitts wird zur Erzeugung des Ladeimpuls ausgenutzt, beispielsweise durch Induktion.

An dieser Stelle sei ausdrücklich erwähnt, dass die Größe (Amplitude) und die Geschwindigkeit der Ummagnetisierungswelle nicht oder nur unwesentlich von der Frequenz des sich verändernden extern erzeugten Magnetfelds abhängt, sondern vorwiegend von den Materialdaten des Magnetisierungsabschnittes. Der Auslösezeitpunkt der Ummagnetisierungswelle hängt davon ab, wann das sich verändernde, extern erzeugte Magnetfeld die genannte Amplitude bzw. Feldstärke erreicht, wobei der Gradient der Änderung des Magnetfeldes bzw. die entsprechende Frequenz keine Rolle spielt. Wird die notwendige Stärke (Amplitude) des Magnetfelds erreicht, beginnt die Blochwand bzw. die Ummagnetisierungswelle zu laufen.

Die Umpolfrequenz bzw. die Veränderung des Magnetfelds spielt nur insoweit eine wenngleich untergeordnete Rolle, weil diese nur über die Anzahl der Initiierungen der Ummagnetisierungswelle Auskunft gibt bzw. sie zeigt lediglich an, wie oft die Ummagnetisierungswelle initiiert wird und damit wie oft ein Ladeimpuls erzeugt wird.

Es ist besonders bevorzugt, dass die als Master agierende Schrittmachereinheit und die als Slave agierende Schrittmachereinheit, besonders bevorzugt alle Schrittmachereinheiten des Schrittmachernetzwerkes, den Ladeimpulserzeugungsabschnitt aufweisen und damit ihre entsprechenden Energiespeicher kontaktlos wieder aufgeladen werden können. Es ist allerdings alternativ denkbar, dass entweder nur die als Master agierende Schrittmachereinheit oder nur die als Slave agierende Schrittmachereinheit den Ladeimpulserzeugungsabschnitt aufweist, beispielsweise in dem erläuterten Fall, dass die als Master agierende Schrittmachereinheit keine Impulse erzeugen muss und sehr energiearm betrieben werden kann.

Vorangehende Ausführungen gelten für die folgenden bevorzugten Ausgestaltungen gleichermaßen.

*Bevorzugt weist der Ladeimpulserzeugungsabschnitt der jeweiligen Schrittmachereinheit(en) mindestens eine Spule auf, die zu dem Magnetisierungsabschnitt derart räumlich angeordnet ist, dass sie bei Auftreten der Ummagnetisierungswelle einen zu dem Ladeimpuls führenden Spannungsimpuls erzeugt.*

Die räumliche Anordnung kann dergestalt sein, dass die Spule um den Magnetisierungsabschnitt gewickelt ist, insbesondere diesen axial umschließend.

Spulen aus elektrisch leitenden Materialen sind bekanntermaßen Induktivitäten. Die Ummagnetisierungswelle führt dazu, dass die Spule aufgrund ihrer induktiven Eigenschaften den zu dem Ladeimpuls führenden Spannungsimpuls erzeugt.

Pro Umpolung des sich verändernden Magnetfelds erzeugt die Spule folglich einen Spannungsimpuls bestimmter Höhe (unabhängig davon, wie schnell sich bzw. mit welcher Frequenz sich das Magnetfeld ändert). Der Magnetisierungsabschnitt und die Spule können beispielsweise so dimensioniert sein, dass der zu dem Ladeimpuls führende Spannungsimpuls in seiner Amplitude 10V und mehr beträgt.

Die von der Spule erzeugten Spannungsimpulse haben alternierend umgekehrte Polaritäten. Zur Ausnutzung aller Spannungsimpulse beinhaltet der Ladeimpulserzeugungsabschnitt bevorzugt eine Ladeelektronik, die die Spannungsimpulse mittels eines Gleichrichters bevorzugt gleichrichtet und/oder in einem Kondensator zwischenspeichert.

Allgemein ausgedrückt besteht der Vorteil darin, dass ein Teil der magnetischen Energie des sich verändernden Magnetfelds in dem Magnetisierungsabschnitt zunächst kumuliert und dann in Form der sich bewegenden Ummagnetisierungswelle quasi schlagartig freigesetzt wird. Die Induktion und damit das Entstehen der elektrischen Spannung findet folglich erst zu diesem Zeitpunkt an/in der Spule statt. D.h. die kontaktlose Energieübertragung beruht nicht darauf, dass das sich verändernde Magnetfeld unmittelbar zur Spannungsinduktion in der Spule verwendet wird, sondern die entsprechende Energie des Magnetfeldes wird in dem Magnetisierungsabschnitt zwischengespeichert und bei Initiierung der Ummagnetisierungswelle quasi schlagartig freigesetzt. Aus diesem Grund kann die Umpolfrequenz so angepasst werden, dass Energie problemlos durch ein aus Metall gebildetes Gehäuse oder Hülle hindurch übertragen werden kann. Es handelt sich um ein Verfahren der indirekten Induktion, d.h. die vom Strom der Primärspule erzeugte Änderung des Magnetflusses führt nicht ausschließlich wie bei der direkten Induktion in der Sekundärspule unmittelbar zu einer Spannung, sondern ein Teil von diesem Fluss wird zunächst in dem Magnetisierungsabschnitt zwischengespeichert. Bei einer bestimmten Feldstärke stimuliert dann der Fluss den Magnetisierungsabschnitt zur Erzeugung einer magnetischen Stoßwelle bestimmter Polarität (Ummagnetisierungswelle) und damit indirekt in der Sekundärspule zu einem Spannungsimpuls bestimmter Polarität mit wesentlich höherer Amplitude. Die Spule des Ladeimpulserzeugungsabschnitts fungiert dabei als die genannte Sekundärspule, die bei Auftreten der Ummagnetisierungswelle den Spannungsimpuls erzeugt. Die genannte Primärspule sitzt beispielsweise in dem im Folgenden noch erläuterten erfindungsgemäßen Ladegerät.

In diesem Punkt bestehen erhebliche Unterschiede zu bekannten kontaktlosen Ladevorgängen für Akkumulatoren, die ein elektromagnetisches Wechselfeld zur Energieübertragung ausnutzen. Ein solches elektromagnetisches Wechselfeld hoher Frequenz ist bei zu implantierenden Schrittmachern kaum oder nur sehr schlecht einsetzbar, weil die Eindringtiefe des elektromagnetischen Wechselfelds in den Körper des Lebewesens und vor allem in ein Metallgehäuse aufgrund von auftretenden Effekten, wie beispielsweise dem Skin-Effekt, zu gering ist.

Der erläuterte Ladeimpulserzeugungsabschnitt, der zumindest in einer der Schrittmachereinheiten, bevorzugt allerdings in der als Master agierenden Schrittmachereinheit und der als Slave agierenden Schrittmachereinheit, enthalten ist, trägt stark dazu bei, dass die Schrittmachereinheiten und damit das gesamte Schrittmachernetzwerk verkleinert werden kann.

Hintergrund hierfür ist die Tatsache, dass die Energiespeicher der entsprechenden Schrittmachereinheiten kontaktlos wieder aufgeladen werden können, ohne mit den erwähnten Problematiken konfrontiert zu sein, die bei einer die direkte Induktion ausnutzenden hochfrequenten kontaktlosen Wiederaufladung auftreten.

Damit entsteht Raum, die Energiespeicher stark zu verkleinern und die jeweilige Elektronik der Schrittmachereinheiten lediglich unter untergeordneter Berücksichtigung ihres Stromverbrauchs aufbauen bzw. auslegen zu können. Beispielsweise könnten die Energiespeicher und/oder eine Leistungsaufnahme der Elektronik bzw. der Einheiten (Sendeeinheit, Empfangseinheit, Detektionseinheit) so ausgelegt/dimensioniert werden, dass der Energieinhalt der Energiespeicher lediglich für wenige Monate, beispielsweise 6 bis 12 Monate, oder Jahre, beispielsweise 1 oder 2 Jahre, ausreicht.

Eine solche Leistungsaufnahme bzw. geringe Laufzeit wäre bei bekannten Schrittmachern, deren Energieversorgung mit einer normalen Batterie gewährleistet wird, ein KO-Kriterium, weil für den Austausch der Batterie notwendige operative Eingriffe eine unannehmbar hohe Frequenz hätten.

*Der Magnetisierungsabschnitt ist bevorzugt durch eine spezielle, z.B. mechanische, Bearbeitung derart aufgebaut, dass die magnetischen Domänen des Magnetisierungsabschnittes gleich ausgerichtet sind. Der Magnetisierungsabschnitt weist bevorzugt einen hartmagnetischen Schalenbereich auf, der einen weichmagnetischen Kernbereich umschließt.*

Der hartmagnetische Schalenbereich entsteht beispielsweise bei der Bearbeitung und Herstellung des Magnetisierungsabschnitts. Ein bevorzugtes Material für den Magnetisierungsabschnitts ist Vicalloy, das beispielsweise in Kaltumformschritten zur Ausrichtung der magnetischen Domänen bearbeitet wird.

*Bevorzugt ist der Magnetisierungsabschnitt mindestens ein Impulsdraht oder ein Wieganddraht. Der Magnetisierungsabschnitt kann auch eine Vielzahl von Impulsdrähten oder eine Vielzahl von Wieganddrähten oder eine Kombination aus mindestens einem Impulsdraht und einem Wieganddraht aufweisen.*

*Die Anzahl der Spulen ist auch nicht auf eine einzige beschränkt. Jedem der Drähte könnte eine eigene Spule zugeordnet sein oder alternativ kann eine Vielzahl der Drähte von einer oder mehreren Spulen umgeben sein. Bevorzugt wird die Spule oder werden die Spulen um einen oder mehrere der Drähte gewickelt.*

In diesem Fall bilden die Spulen jeweils eine Sekundärspule des erläuterten Verfahrens indirekter Induktion, zu der Energie mittelbar über den Magnetisierungsabschnitt von der Primärspule, die bevorzugt in dem noch zu erläuternden Ladegerät sitzt, übertragen wird.

*Bevorzugt ist das erfindungsgemäße Schrittmachernetzwerk so ausgebildet, dass die Elektronik der als Master agierenden Schrittmachereinheit und*/*oder der als Slave agierenden Schrittmachereinheit zusammen mit dem jeweiligen Energiespeicher und dem jeweiligen Ladeimpulserzeugungsabschnitt von einer Hülle bzw. einem Gehäuse vollständig umgeben ist, die*/*das aus einem Material gebildet ist, das von dem Körper des Lebewesens nicht abgestoßen wird. Das Material ist bevorzugt ein nicht-ferromagnetisches Metall, insbesondere Titan, oder eine, insbesondere Titan umfassende, Metalllegierung.*

Alternative Metalle sind Edelstähle.

Die elektrische Leitfähigkeit der genannten Materialien ist von Bedeutung, um hochfrequente Störfelder, die die Funktion des Schrittmachernetzwerkes, beispielsweise mit der Funktionsweise eines Zweikammer-Herzschrittmachers, beeinträchtigen können, zu dämpfen. Auch kann das elektrisch leitende Gehäuse als Massekontakt für den Stromkreis vorgesehen sein. Wenn das Gehäuse aus einem nicht leitenden Material gebildet ist, kann eine gesonderte Masseelektrode vorgesehen sein

Der Elektrodenabschnitt ist beispielsweise lösbar an der Hülle bzw. dem Gehäuse befestigt bzw. durchläuft diese(s), wobei er gegenüber der Hülle/dem Gehäuse bevorzugt isoliert ist, und ist innerhalb der Hülle/Gehäuse mit der Elektronik verbunden. Alternativ kann der Elektrodenabschnitt durch auf einer Außenoberfläche freiliegende Elektrodenflächen, die an dem entsprechenden Körperabschnitt anliegen, gebildet sein.

Die äußere Gestalt der Schrittmachereinheit(en) kann dergestalt sein, dass das Gehäuse die genannten Elemente aufnimmt, und der Elektrodenabschnitt an einer Seite des Gehäuses vorsteht oder freiliegt. Die Funktionalität der kontaktlosen Wiederaufladung ermöglicht eine so starke Verkleinerung des Gehäuses und des Elektrodenabschnittes, dass die entsprechende Schrittmachereinheit potenziell auch in ein menschliches Herz, d. h. in einen Innenraum des Vorhofs oder der Kammer, geschoben und dort über ihren Elektrodenabschnitt an dem entsprechenden Körperabschnitt verankert werden kann oder dort kontaktierend anliegt.

Ein bevorzugtes Volumen des Gehäuses liegt beispielsweise in der Grö-Benordnung von unter 1, 2, 3, 4, 5, 6, 7 oder 8 cm³; beispielsweise hat das Gehäuse eine Kugelform mit 1 cm Durchmesser. Alternativ kann das Gehäuse eine zylindrische Form mit einem Durchmesser von beispielsweise 1 oder 0,5 cm und einer Höhe von beispielsweise 2,5 cm haben. Die Länge des Elektrodenabschnitts kann eine Länge von wenigen Zentimetern mithin von unter 1 cm, d. h. wenigen Millimetern, betragen. Bevorzugt besitzt der Elektrodenabschnitt keine Kunststoffisolierung und wird bei bestimmungsgemäßer Implantation annähernd vollständig in dem entsprechenden Körperabschnitt so verankert, dass das Gehäuse an dem Körperabschnitt anliegt.

Die bereits erwähnte Tatsache, dass der Magnetisierungsabschnitt die magnetische Energie kumuliert und erst die Freisetzung in Form der Ummagnetisierungswelle zu dem Entstehen der induzierten elektrischen Spannung bzw. dem Spannungsimpuls innerhalb des Gehäuses bzw. der Hülle führt, schafft Freiräume für die Wahl des bevorzugt nicht-ferromagnetischen Materials der genannten Hülle bzw. des Gehäuses, weil keine unmittelbaren Anforderungen an Übertragungsfrequenzen gestellt werden müssen.

*Bevorzugt beinhaltet der Ladeimpulserzeugungsabschnitt der Schrittmachereinheit(en) in einer Richtung, in der die mindestens eine Spule gewickelt ist oder in der die Spulen gewickelt sind, an mindestens einem Endabschnitt des Magnetisierungsabschnittes eine magnetische Sammellinse zur Bündelung und Führung des sich verändernden, extern erzeugten Magnetfeldes auf den Magnetisierungsabschnitt.*

Die genannte Richtung entspricht der Längsrichtung der Spule bzw. der Spulen, in der sie gewickelt ist bzw. sind. Bevorzugt sind an beiden Endabschnitten des Magnetisierungsabschnitts jeweils mindestens eine magnetische Sammellinse angeordnet, die das sich verändernde, extern erzeugte Magnetfeld auf dem Magnetisierungsabschnitt bündeln bzw. zu diesem leiten.

Alternativ zur Verwendung eigenständiger Sammellinse(n) besteht auch die Möglichkeit, dass die Hülle bzw. das Gehäuse der als Master agierenden Schrittmachereinheit und/oder der als Slave agierenden Schrittmachereinheit gezielt teilweise oder abschnittsweise aus einem ferromagnetischen Material gebildet ist oder teilweise oder abschnittsweise mit einem solchen beschichtet ist und, z.B. durch eine Teilung in zwei getrennte Hälften so aufgebaut ist, dass es die Funktion der magnetischen Sammellinsen direkt übernimmt. Durch die dann größere Ausgestaltung der Sammellinsen kann das vom Ladegerät zu erzeugende Magnetfeld weiter reduziert werden.

*Die mindestens eine magnetische Sammellinse der Schrittmachereinheit(en) ist bevorzugt aus einem ferromagnetischen Metall gebildet, welches das extern erzeugte Magnetfeld für den Magnetisierungsabschnitt bündelt.*

Die magnetische(n) Sammellinse(n) ist/sind beispielsweise aus Ferrit ausgebildet und haben beispielsweise die Form eines Hohlzylinders, dessen Achse in Richtung des jeweiligen Endabschnitts des Magnetisierungsabschnittes weist.

Der Magnetisierungsabschnitt ist bevorzugt in den Hohlzylinder eingeführt.

Die Verwendung der magnetischen Sammellinse(n) ermöglicht beispielsweise, dass das im Folgenden noch zu erläuternde Ladegerät ein schwächeres Magnetfeld erzeugen muss und dass dessen Ausrichtung in Bezug auf die Schrittmachereinheit(en) weniger kritisch ist.

*Die Elektronik* der als Master agierenden Schrittmachereinheit und/oder der als Slave agierenden Schrittmachereinheit *beinhaltet bevorzugt keine Elemente aus ferromagnetischen Materialien und*/*oder der Ladeimpulserzeugungsabschnitt des erfindungsgemäßen elektronischen Schrittmachers beinhaltet bis auf den Magnetisierungsabschnitt und, wenn bevorzugt vorgesehen, die mindestens eine magnetische Sammellinse keine Elemente aus ferromagnetischen Materialen.*

Diese Ausgestaltung der als Master agierenden Schrittmachereinheit und/oder der als Slave agierenden Schrittmachereinheit ist dahingehend vorteilhaft, dass die nicht aus ferromagnetischen Materialien gebildeten Elemente durch das extern erzeugte Magnetfeld nicht beeinträchtigt bzw. gestört werden.

*Weiterhin bevorzugt ist die Elektronik der als Master agierenden Schrittmachereinheit und*/*oder der als Slave agierenden Schrittmachereinheit eingerichtet, ein Signal auszusenden, welches die Qualität des Ladeimpulses anzeigt.*

Das genannte Signal kann beispielsweise ein niederfrequentes Signal sein, das den Köper des Lebewesens und, wenn keine Antenne hierfür vorgesehen ist, die Hülle bzw. das Gehäuse des erfindungsgemäßen Schrittmachers durchdringt.

Wenn die als Master agierende Schrittmachereinheit die erläuterte Sendeeinheit aufweist, kann das die Qualität anzeigende Signal von dieser versendet werden.

Die Qualität des Ladeipulses verhält sich proportional zum Wert des Integrales (*∫ i dt*)*.* Bei guten Ladeimpulsen, d.h. mit sehr hoher Qualität beträgt der Wert beispielsweise 100nC.

Die Qualität der Ladeimpulse kann beispielsweise daran abgeleitet werden, wie stark aufeinanderfolgende Ladeimpulse bei Veränderung des externen Magnetfeldes schwanken, d.h. wie ihre Strom- und/oder Spannungsamplituden schwanken, und/oder wie ihre zeitlichen Breiten schwanken.

Weiterhin beispielsweise kann das die Qualität angebende Signal den Wert des Integrals des Stroms des Ladeimpulses über der Zeit (*∫ i dt*)*,* d.h. seinen Ladungsinhalt, angeben.

Das die Qualität des Ladeimpulses angebende Signal kann alternativ beispielsweise ein binäres Signal sein, das einen OK-Zustand einnimmt, wenn der Ladeimpuls, bzw. sein Ladungsinhalt, einen Schwellenwert übersteigt, und einen NG-Zustand einnimmt, wenn der Ladeimpuls den Schwellenwert nicht übersteigt. Der Schwellenwert kann beispielsweise bei 50%, 55%, 60%, 65%, 70%, 75%, 80%, 85% oder 90% des durch den Magnetisierungsabschnittes lieferbaren Ladungsinhaltes liegen. Als Beispiel können Werte von 50nC, 55nC, 60nC, 65nC, 70nC, 75nC, 80nC, 85nC oder 90nC genannt werden.

Die Elektronik kann diesbezüglich derart eingerichtet sein, dass sie das die Qualität angebende Signal für jeden Ladeimpuls aussendet oder alternativ nur für solche der Ladeimpulse die in bestimmten Intervallen hintereinander auftreten. Wenn das externe Magnetfeld von einem im Folgenden näher erläuterten Ladegerät erzeugt wird, das das Magnetfeld mit einer Umpolfrequenz im kHz-Bereich erzeugt, kann die Elektronik das die Qualität angebende Signal beispielsweise für solche der Ladeimpulse erzeugen, die in Intervallen von beispielsweise >1, 25, 50, 100, 200, 500, 750, oder 1000 Ladeimpulsen auftreten.

Die Sendeeinheit der als Master agierenden Schrittmachereinheit und die Empfangseinheit der als Slave agierenden Schrittmachereinheit, die beide im Vorhergehenden erläutert wurden, können beide bevorzugt eine Einheit mit Sende- und Empfangsfunktionen sein, und damit beispielsweise als Schnittstelle zur Programmierung der Schrittmachereinheit(en) dienen. In diesem Zusammenhang können die Schrittmachereinheiten hierrüber derart umprogrammiert werden, dass sie vollständig autark voneinander arbeiten, d.h. jede Schrittmachereinheit überwacht die Eigenaktion des Körperabschnittes eigenständig und erzeugt je nach Bedarf den Impuls eigenständig. Ferner könnten die Schrittmachereinheiten hierüber ein- oder ausgeschaltet werden.

Außerdem kann die entsprechende Elektronik so ausgestaltet sein, dass ein Ladezustand der entsprechenden Energiespeicher über die Sende- und Empfangsfunktion der als Master und/oder der als Slave agierenden Schrittmachereinheit abgefragt werden kann.

Als alternative zu dem die Qualität angebenden Signal kann das im Folgenden erläuterte Ladegerät so ausgestaltet sein, dass es durch die genannten Funktionen den Ladezustand des/der Energiespeicher(s) in bestimmten zeitlichen Abständen abfragt und auf Basis der Informationen über eine Änderung des Ladezustandes bzw. der Ladezustände, den zeitlichen Abstand und der im Folgenden noch genannten Umpolfrequenz einen Rückschluss auf die Qualität der einzelnen Ladeimpulse zieht. Der zeitliche Abstand beträgt bevorzugt 0,5min, 1,0min, 1,5min, 2,0min, 2,5min, 3,0min, 3,5min, 4,0min, 4,5min, 5,0min.

*Die Erfindung betrifft ebenfalls ein Ladegerät für ein Schrittmachernetzwerk, wobei das Ladegerät eingerichtet ist, ein sich mit einer Umpolfrequenz und Amplitude veränderndes Magnetfeld zu erzeugen. Das Ladegerät wird bei bestimmungsgemäßer Verwendung vorübergehend auf einer Körperoberfläche des Lebewesens oder in der Nähe der Körperoberfläche des Lebewesens derart angeordnet, dass das Magnetfeld in den Körper und die als Master agierende Schrittmachereinheit und*/*oder die als Slave agierende Schrittmachereinheit zur Beeinflussung des entsprechenden Ladeimpulserzeugungsabschnittes eindringt.*

*Die Umpolfrequenz liegt bevorzugt in einem Bereich von*
- *X bis 10kHz, wobei X>0 und X>= 0,1kHz, 0,2kHz, 0,3kHz,..., 4,9kHz,...,9,9kHz ist.*

Das Ladegerät beinhaltet beispielsweise eine oder eine Vielzahl von Spulen, die in dem im Vorhergehenden erwähnten Verfahren indirekter Induktion als die Primärspule(n) fungieren. Bevorzugt ist ein Kern, beispielsweise aus Ferrit, in die Spule(n) eingesetzt.

Das Ladegerät erzeugt bestimmungsgemäß einen Stromfluss durch die Spule(n) für den Aufbau eines sich verändernden elektromagnetischen Feldes, das sich mit der erwähnten Umpolfrequenz umpolt. Wenn das Ladegerät auf der Körperoberfläche bzw. in deren Nähe angeordnet ist, kann das Wechselfeld in den Körper des Lebewesens und die Schrittmachereinheit(en) eindringen.

Der magnetische Anteil des sich verändernden elektromagnetischen Feldes bildet das im Vorhergehenden erläuterte, extern erzeugte Magnetfeld, das den Magnetisierungsabschnitt zur Initiierung der Ummagnetisierungswelle beeinflusst.

Die Stärke und/oder die Umpolfrequenz des elektromagnetischen Wechselfeldes können bevorzugt in dem Ladegerät gesteuert werden. Dies ist dahingehend vorteilhaft, dass das Ladegerät in Abhängigkeit von der Lage der Schrittmachereinheiten innerhalb des Körpers bzw. in Abhängigkeit von der notwendigen Eindringtiefe eingestellt werden kann.

*Bevorzugt beinhaltet das erfindungsgemäße Ladegerät eine Vielzahl von Spulen zur Erzeugung des sich verändernden Magnetfeldes, wobei die Vielzahl von Spulen auf Basis des*/*der die Qualität des Ladeimpulses*/*der Ladeimpulse anzeigenden Signale, bspw. deren Absolutwerte und*/*oder deren Änderung, für eine Optimierung des Ladeimpulses*/*der Ladeimpulse entsprechend angesteuert werden können.*

*Alternativ beinhaltet das erfindungsgemäße Ladegerät eine Vielzahl von Spulen zur Erzeugung des sich verändernden Magnetfeldes, wobei*
*das Ladegerät eingerichtet ist, (i) den Ladezustand des Energiespeichers der als Master und*/*oder der als Slave agierenden Schrittmachereinheit in bestimmten zeitlichen Abständen abzufragen und auf Basis der Änderung des*/*der Ladezustände, des zeitlichen Abstandes und der Umpolfrequenz einen Rückschluss auf die Qualität der Ladeimpulse zu ermitteln, und (ii) die Vielzahl von Spulen auf Basis des ermittelten Rückschlusses entsprechend anzusteuern.*

Die Spulen der Vielzahl von Spulen sind bevorzugt so räumlich angeordnet, dass das Ladegerät durch die Ansteuerung der Spulen die Ausrichtung des erzeugten Magnetfeldes ändern kann. Dies hat den Vorteil, dass das Ladegerät die Ausrichtung des Magnetfeldes unter Berücksichtigung des/der die Qualität des Ladeimpulses/der Ladeimpulse angebenden Signale (bspw. deren Absolutwerte und/oder deren Änderung) oder unter Berücksichtigung des ermittelten Rückschlusses ändern kann, um die Qualität der Ladeimpulse zu verbessern bzw. zu optimieren.

Das Ladegerät ist bevorzugt eingerichtet, die Spulen zur Ausrichtung des Magnetfeldes automatisch anzusteuern.

Im Folgenden wird eine bevorzugte Ausführungsform der Erfindung unter Bezug auf die beigefügte Figur erläutert.
**Figur 1** zeigt eine schematische Darstellung eines erfindungsgemäßen Implantat-Schrittmachers ;
**Figur 2** zeigt eine schematische Darstellung eines entsprechenden Ladegerätes;
**Figur 3** zeigt eine alternative Ausgestaltung des in Figur 2 gezeigten Ladegerätes mit einer Vielzahl von Spulen.

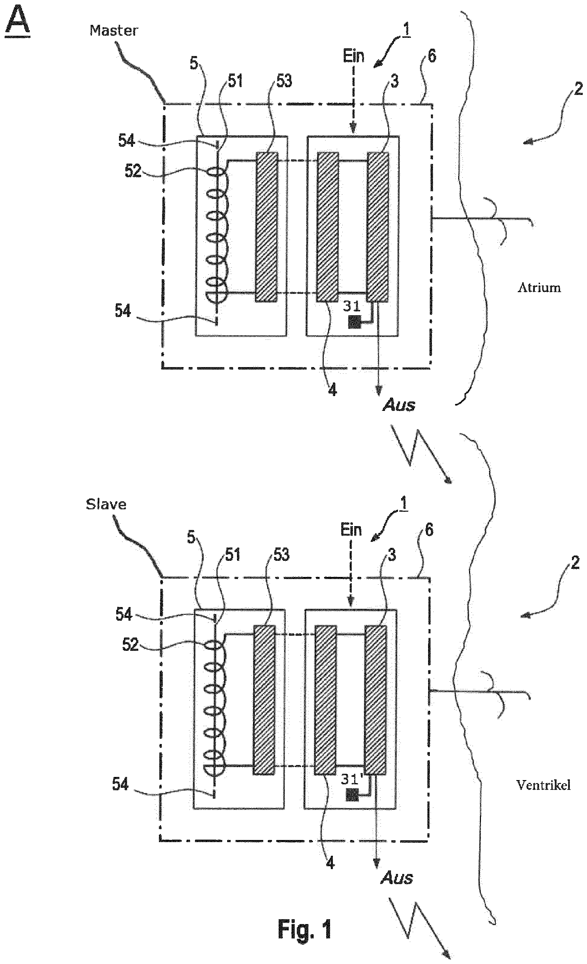

Figur 1 zeigt den schematischen Aufbau eines erfindungsgemäßen Schrittmachernetzwerkes A, das eine Vielzahl von elektronischen Schrittmachereinheiten beinhaltet. In dem erfindungsgemäßen Schrittmachernetzwerk A agiert zumindest eine der Schrittmachereinheiten als ein Master und die verbleibenden Schrittmachereinheiten als Slave, deren Verhalten sich nach dem des/der Master richten.

Das Schrittmachernetzwerk A beinhaltet in dieser bevorzugten Ausführungsform die als Master agierende Schrittmachereinheit 1 und eine einzige als Slave agierende Schrittmachereinheit 1'.

Das Schrittmachernetzwerk A fungiert in dieser bevorzugten Ausführungsform als ein Zweikammer-Herzschrittmacher, der bestimmungsgemäß vollständig in den menschlichen Körper implantiert wird.

Die als Master agierende Schrittmachereinheit 1 befindet sich bevorzugt in einem Innenraum des Vorhofs (Atrium) des menschlichen Herzen oder an dessen Außenseite und ist mit einem Elektrodenabschnitt 2 dort in einem Wandabschnitt des menschlichen Herzen (erster Körperabschnitt) verankert. Die als Slave agierende Schrittmachereinheit 1' sitzt hingegen bevorzugt in einer Kammer (Ventrikel) des menschlichen Herzen oder an dessen Außenseite und ist einem entsprechenden Elektrodenabschnitt 2' dort gleichermaßen in einem Wandabschnitt (zweiter Körperabschnitt) des menschlichen Herzen verankert.

Die mit identischen Bezugszeichen versehenen Elemente der als Master agierenden Schrittmachereinheit 1 und der als Slave agierenden Schrittmachereinheit 1' sind identisch aufgebaut und werden nur einmalig unter Bezug auf die als Master agierende Schrittmachereinheit 1 erläutert:
Der Elektrodenabschnitt 2 ist mit einer Elektronik 3 verbunden.

Die Elektronik 3 ist eingerichtet, die notwendigen Funktionen der als Master agierenden Schrittmachereinheit 1 zu übernehmen. Die Elektronik 3 erhält ein Eingangssignal Ein(Körperdaten), über das die Schrittmachereinheit 1 bzw. die Elektronik 3 erkennen kann, ob die zu überwachende und steuernde Funktion stimuliert bzw. gesteuert werden muss. Das Eingangssignal Ein (Körperdaten) gewinnt die Elektronik entweder über den Elektrodenabschnitt 2 oder über eine gesonderte, nicht gezeigte, Überwachungselektrode.

Wenn die Elektronik 3 beispielsweise erkennt, dass nach Ablauf eines bestimmten Zeitintervalls keine Eigenaktion des Vorhofs (Atrium) vorliegt, erzeugt sie einen Impuls bzw. Stimulationsimpuls (Strom- und/oder Spannungsimpuls), den sie zur Anregung des Vorhofs (Atrium) über den Elektrodenabschnitt 2 abgibt.

Die Elektronik 3 ist bevorzugt so eingerichtet, dass sie nur im Bedarfsfall den Impuls erzeugt und den Vorhof (Atrium) anregt, d. h., wenn die Elektronik 3 feststellt, dass bis Ablauf des bestimmten Zeitintervalls eine Eigenaktion vorliegt, verhält sie sich passiv und erzeugt keinen Impuls.

Der Herzschrittmacher 1 beinhaltet zur Versorgung der Elektronik 3 einen elektrischen Energiespeicher 4, beispielsweise einen Akkumulator, der mit der Elektronik 3 elektrisch verbunden ist. Der Energiespeicher 4 ist beispielsweise ein Lithium-Ionen-Akkumulator, der wieder aufgeladen werden kann. Eine andere Lösung für einen Energiespeicher wäre z.B. ein Kondensator mit extrem geringer Selbstentladung (z.B. Gold-Cap).

Die als Master agierende Schrittmachereinheit 1 beinhaltet weiterhin einen Ladeimpulserzeugungsabschnitt 5, über den der Energiespeicher 4 wieder aufgeladen werden kann. Der Ladeimpulserzeugungsabschnitt 5 ermöglicht ein kontaktloses Laden des Energiespeichers 4.

Der Ladeimpulserzeugungsabschnitt 5 beinhaltet als wesentliche Elemente mindestens einen Impulsdraht oder Wieganddraht 51, der von einer Spule 52 axial umschlossen wird bzw. um den die Spule 52 gewickelt ist, und eine Ladeelektronik 53.

Der Impulsdraht oder Wieganddraht 51 bildet einen Magnetisierungsabschnitt, der durch ein sich veränderndes, extern erzeugtes Magnetfeld beeinflusst werden kann. Bevorzugt kann der Magnetisierungsabschnitt 51 eine Vielzahl von Impulsdrähten und/oder Wieganddrähten beinhalten, wobei jeder der Drähte oder eine Vielzahl der Drähte von einer oder mehreren Spulen umschlossen sein kann.

Das sich verändernde Magnetfeld wird beispielsweise durch ein im Folgenden noch erläutertes Ladegerät erzeugt.

Der Magnetisierungsabschnitt 51 weist gleichmäßig ausgerichtete magnetische Domänen auf, die bei Veränderung des Magnetfeldes ab einer bestimmten Amplitude bzw. Feldstärke in der Größenordnung weniger MilliTesla beginnen, sich umzumagnetisieren (umzuklappen). Dies führt dazu, dass physikalisch gesehen eine Ummagnetisierungswelle (Blochwand) über den Magnetisierungsabschnitt läuft. In der Literatur wird dieses Ereignis auch als großer Barkhausen-Sprung bezeichnet.

Die Größe und Geschwindigkeit der Ummagnetisierungswelle ist unabhängig von der Frequenz (Umpolfrequenz), mit der sich das extern erzeugte Magnetfeld ändert. Die über den Magnetisierungsabschnitt laufende Ummagnetisierungswelle erzeugt in der/den um den Magnetisierungsabschnitt 51 gewickelte(n) Spule(n) 52 einen Spannungsimpuls.

Der Spannungsimpuls wird bevorzugt von der Ladeelektronik 53 verarbeitet. Die Ladeelektronik 53 beinhaltet beispielsweise einen Gleichrichter zur Gleichrichtung der Spannungsimpulse der Spule(n), die alternierend mit jeweils umgekehrter Polarität erzeugt werden, und bevorzugt einen Kondensator (beispielsweise auch ein Gold-Cap) zur Zwischenspeicherung elektrischer Energie.

Die Ladeelektronik 53 gibt letztendlich einen Ladeimpuls an den Energiespeicher 4 aus, wodurch dieser aufgeladen wird.

Die Elektronik 3 kann bevorzugt ausgestaltet sein, ein Signal *Aus* auszugeben, das die Qualität des von der Ladeelektronik 53 ausgegebenen Ladeimpulses angibt. Beispielsweise erfasst die Elektronik 3 die Stärke des Ladeimpulses und generiert hierauf beruhend das Signal *Aus.* Die Ausgabe des Signals *Aus* erfolgt beispielsweise als niederfrequentes Funksignal. Das Signal *Aus* wird von dem im Folgenden noch erläuterten Ladegerät verarbeitet.

Die Elektronik 3, der Energiespeicher 4 und der Ladeimpulserzeugungsabschnitt 5 sind zusammen in ein Gehäuse 6 aufgenommen und werden von diesem vollständig umschlossen. Das Gehäuse 6 ist bevorzugt aus Titan bzw. einer entsprechenden Legierung aufgebaut und eignet sich deshalb hervorragend zur Implantation in den menschlichen Körper, weil keine Abstoßungsreaktionen auftreten und hält als metallischer Körper hochfrequente Störfelder fern. Ein metallischer Körper kann zudem als die für den Stromimpuls notwendige Masseelektrode verwendet werden, was z.B. bei einem Glaskörper nicht möglich wäre. Die Ausbildung des Gehäuses 6 als Glaskörper ist allerdings nicht ausgeschlossen, in diesem Fall beinhaltet die als Master agierende Schrittmachereinheit 1 eine gesonderte, in Figur 1 nicht gezeigte, Masseelektrode.

Die erläuterten Elemente sind in der als Slave agierenden Schrittmachereinheit 1' ebenfalls enthalten und identisch aufgebaut. D. h. die als Slave agierende Schrittmachereinheit 1' erhält ein Eingangssignal Ein über ihren Elektrodenabschnitt 2 oder eine gesonderte Überwachungselektrode zur Feststellung, ob eine Eigenaktion der Kammer (Ventrikel) vorliegt oder nicht. Bei Bedarf kann die Elektronik 3 der als Slave agierenden Schrittmachereinheit 1' ihren Impuls erzeugen und an die Kammer (Ventrikel), die einem zweiten Körperabschnitt entspricht, abgeben. Gleichermaßen wie der Energiespeicher der als Master agierenden Schrittmachereinheit 1 kann der Energiespeicher der als Slave agierenden Schrittmachereinheit 1' kontaktlos wieder aufgeladen werden. Hierfür beinhaltet die als Slave agierende Schrittmachereinheit 1' den bereits erläuterten Ladeimpulserzeugungsabschnitt.

Die als Master agierende Schrittmachereinheit 1 und die als Slave agierende Schrittmachereinheit 1' bilden zusammen ein Schrittmachernetzwerk, in dem sie zur Abbildung der Funktion des Zweikammer-Herzschrittmachers zusammenwirken.

Hierfür beinhaltet die als Master agierende Schrittmachereinheit 1 eine Sendeeinheit 31. Die Elektronik 3 ist eingerichtet, eine Information über die Sendeeinheit 31 zu versenden, wobei die Informationen den Inhalt besitzt, dass eine Eigenaktion des Vorhofs vorliegt, oder, dass der Impuls an den Vorhof abgegeben wurde, weil keine Eigenaktion des Vorhofs durch die Elektronik festgestellt werden konnte. Die Sendeeinheit 31 kann beispielsweise auf dem Funkstandard Bluetooth oder Low Energy Bluetooth arbeiten; alternativ kann die Sendeeinheit auch in einem Bereich niedriger Frequenzen arbeiten.

Ein Unterschied in der Funktionsweise der als Slave agierenden Schrittmachereinheit 1' zu der der als Master agierenden Schrittmachereinheit 1 besteht darin, dass sie im Normalfall nicht autark, sondern basierend auf der versendeten Information agiert. Hierfür beinhaltet die als Slave agierende Schrittmachereinheit 1' eine Empfangseinheit 31', über die sie die versendete Information empfängt.

Die Elektronik 3 der als Slave agierenden Schrittmachereinheit 1' startet bevorzugt basierend auf der empfangenen Information eine Zeitmessung, die beispielsweise einem A/V-Intervall (normales Zeitintervall zwischen den Eigenaktionen des Vorhofs und der Kammer) eines gesunden Herzen entspricht. Wenn die Elektronik 3 der als Slave agierenden Schrittmachereinheit 1' auf Basis des Signals Ein bis zu dem Ablauf des A/V-Intervalls keine Eigenaktion der Kammer feststellen kann, erzeugt sie einen Impuls und gibt diesen über ihren Elektrodenabschnitt 2 an die Kammer (Ventrikel) ab. Stellt sie hingegen auf Basis des genannten Signals eine Eigenaktion der Kammer (Ventrikel) fest, verhält sie sich passiv, ergo gibt keinen Impuls ab.

Die Sendeeinheit 31 der als Master agierenden Schrittmachereinheit 1 und die Empfangseinheit 31' der als Slave agierenden Schrittmachereinheit können beide bevorzugt Sende- und Empfangsfunktionen aufweisen, und damit als Schnittstelle zur Programmierung der Schrittmachereinheit(en) dienen.

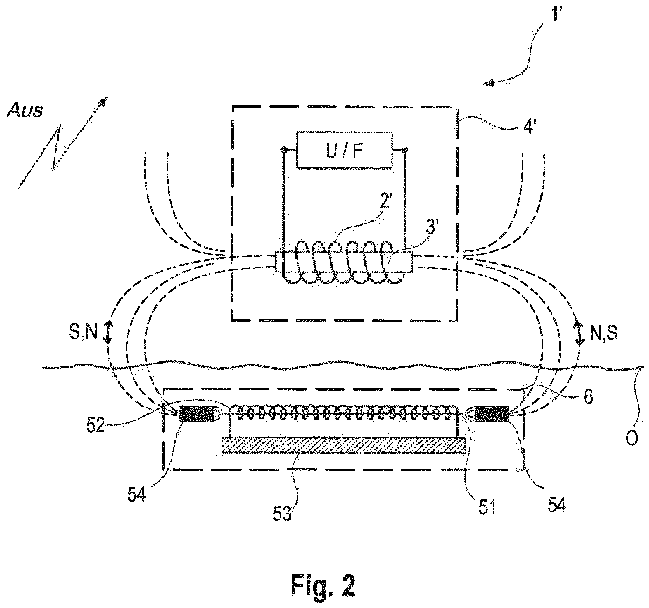

Figur 2 zeigt schematisch den Aufbau eines erfindungsgemäßen Ladegerätes 1' das zum Wiederaufladen der Schrittmachereinheiten 1, 1' dient.

Die Schrittmachereinheiten nutzen ein Verfahren indirekter Induktion um den Energiespeicher 4 wieder aufzuladen, d.h. die Energie wird nicht wie bei der direkten Induktion direkt von einer Primär- unverzögert zu einer Sekundärspule übertragen (Transformatorprinzip), sondern indirekt von einer Primärspule, die in dem folgend erläuterten Ladegerät sitzt, erst auf den die Energie speichernden Magnetisierungsabschnitt 51 und von dort verzögert zu der/den den Magnetisierungsabschnitt 51 umschließenden Spulen 52.

Das Ladegerät 1' beinhaltet zum Beispiel eine Spule (Primärspule) 2', deren Strom bzw. Spannung in Amplitude und Frequenz geregelt werden kann. Bevorzugt hat die Spule 2' zur Feldverstärkung einen ferromagnetischen Kern 3', z.B. aus Ferrit.

Ein Gehäuse 4' nimmt die entsprechenden Komponenten des Ladegerätes 1' auf. Das Gehäuse 4' wird bei bestimmungsgemäßer Verwendung zweitweise in der Nähe oder auf einer Oberfläche O des menschlichen Körpers so angeordnet, dass das von der Primärspule 2' im Ladegerät erzeugte externe Magnetfeld die Magnetisierungsabschnitte 51 der Schrittmachereinheiten erreicht.

Bei Betrieb des Ladegeräts 1' erzeugt dieses über die Primärspule 2' ein sich veränderndes Magnetfeld, das einen Anteil des von der Primärspule 2' erzeugten elektromagnetischen Feldes bildet. Das erzeugte sich verändernde Magnetfeld polt sich mit einer bestimmten Umpolfrequenz um und erreicht die Magnetisierungsabschnitte 51 der Schrittmachereinheiten. Jede Umpolung führt ab Erreichen einer bestimmten Feldstärke zu der Initiierung der Ummagnetisierungswelle, wobei die Spule 52, die in dem erwähnten Verfahren der indirekten Induktion die Sekundärspule bildet, alternierend positive und negative Spannungsimpulse erzeugt.

Die Spannungsimpulse werden, wie bereits erläutert, von der Ladeelektronik 53 der Schrittmachereinheiten verarbeitet, sodass die Ladeelektronik 53 letztendlich den Ladeimpuls an den Energiespeicher 4 abgibt.

Ein wesentlicher Punkt ist, wie bereits erwähnt, dass die kontaktlose Energieübertragung nicht darauf beruht, dass das sich verändernde Magnetfeld, das von dem Ladegerät 1' erzeugt wird, direkt zur Spannungsinduktion in der Sekundärspule 52 verwendet wird, sondern indirekt, indem die entsprechende Energie des Magnetfeldes in dem Magnetisierungsabschnitt 51 zwischengespeichert und dann bei Initiierung der Ummagnetisierungswelle quasi schlagartig freigesetzt wird, wodurch der Spannungsimpuls in der Sekundärspule 52 durch Induktion erzeugt wird. Aus diesem Grund kann die Umpolfrequenz so angepasst werden, dass die Energie problemlos auch durch das aus Metall gebildete Gehäuse 6 hindurch übertragen werden kann.

Die Stärke und/oder Umpolfrequenz des von der Primärspule 2' erzeugten Magnetfeldes bzw. elektromagnetischen Feldes kann in dem Ladegerät 1' gesteuert werden, um das Wiederaufladen des Energiespeichers 4 der Schrittmachereinheiten an die spezifische Lage der einzelnen Schrittmachereinheiten 1 in dem Körper des Lebewesens bzw. die notwendige Eindringtiefe anzupassen und die Ladezeit zu minimieren. Die Steuerung der Stärke und/oder Umpolfrequenz des von der Primärspule 2' erzeugten Magnetfeldes bzw. elektromagnetischen Feldes erfolgt in dem Ladegerät 1' bevorzugt auf Basis der Signale *Aus,* die von den Schrittmachereinheiten ausgesendet werden. Hierfür beinhaltet das Ladegerät 1' entsprechende Empfangseigenschaften, um die Funk-Signale *Aus* zu empfangen.

Bevorzugt beinhalten die Schrittmachereinheiten in der Längsrichtung der Spule(n) 52 an Endabschnitten ihres jeweiligen Magnetisierungsabschnittes 51 magnetische Sammellinsen 54 zur Bündelung des sich verändernden Magnetfeldes. Die magnetischen Sammellinsen 54 können bevorzugt die Form eines Hohlzylinders haben, in den der Impulsdraht/Wieganddraht bzw. die Impulsdrähte/Wieganddrähte eingeführt sind.

Alternativ oder zusätzlich zu den magnetischen Sammellinsen 54 kann das jeweilige Gehäuse 6 der Schrittmachereinheiten aus zwei zusammengesetzten Gehäuseabschnitten aufgebaut sein. Die Gehäuseabschnitte können aus ferromagnetischen Metallen ausgebildet sein oder mit solchen beschichtet sein, wobei die Ausrichtung des Ladeimpulserzeugungsabschnittes 5 innerhalb des Gehäuses 6 so gewählt ist, dass die Gehäuseabschnitte als zusätzliche oder auch alleinige Sammellinsen 54 wirken.

Pro Umpolung des sich verändernden externen Magnetfeldes wird die Ummagnetisierungswelle, die über den Magnetisierungsabschnitt 51 läuft initiiert und letztendlich einer der Ladeimpulse zum Wiederaufladen des Energiespeichers 4 erzeugt.

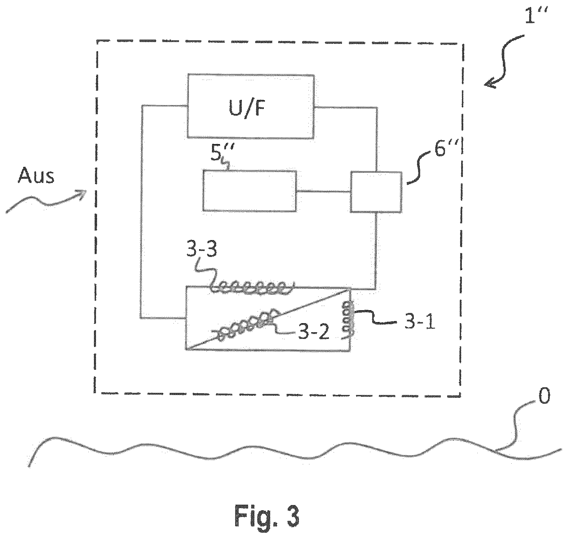

Figur 3 zeigt eine alternative Ausgestaltung eines erfindungsgemäßen Ladegeräts 1".

Die Schrittmachereinheiten sind, wie in Figur 2 anhand der als Master agierenden Schrittmachereinheit 1 beispielhaft gezeigt, angeordnet, aber in Figur 3 nicht mehr gezeigt.

Das gezeigte Ladegerät 1" unterscheidet sich von dem aus Figur 2 lediglich dadurch, dass eine Vielzahl von Spulen 3-1, 3-2, 3-3, die jeweils als die genannte Primärspule fungieren, vorgesehen ist. Das Ladegerät 1" beinhaltet bevorzugt eine Elektronik 5" und einen Multiplexer 6". Die Elektronik 5" ist dazu eingerichtet, den Multiplexer 6" anzusteuern und hierdurch festzulegen, welche oder in welcher Kombination die Spulen 3-1, 3-2, 3-3 zur Erzeugung des Magnetfeldes verwendet werden. Die Ansteuerung der Spulen 3-1, 3-2, 3-3 beruht auf den (Funk)signale *Aus* der Schrittmachereinheiten, die die Qualität der Ladeimpulse angeben.

Die Spulen 3-1, 3-2, 3-3 sind räumlich unterschiedlich angeordnet, wodurch die Orientierung des Magnetfeldes zur Verbesserung und Optimierung des Ladeimpulses geändert werden kann.

Jede der Spulen 3-1, 3-2, 3-3 beinhaltet bevorzugt einen Kern wie er in Figur 2 gezeigt und in die Spule 2' eingeführt ist.

Durch das erfindungsgemäße Schrittmachernetzwerk können die Funktionen eines Mehrkammer-Herzschrittmachers, beispielsweise des Zweikammer-Herzschrittmachers, realisiert werden. Gleichzeitig sind die Elektrodenabschnitte 2 sehr kurz ausgebildet und benötigen beispielsweise keine Kunststoffisolierung, die zu gesundheitlichen Problemen führen könnte.

Darüber hinaus können die Schrittmachereinheiten aufgrund ihrer Funktionalität der kontaktlosen Wiederaufladung problemlos so klein ausgebildet werden, dass sie direkt im oder am Herzen angeordnet werden können. Das Gehäuse 6 der Schrittmachereinheiten 1, 1' hat beispielsweise ein Volumen von kleiner/gleich 1 cm³ und ein Gewicht von wenigen Gramm, beispielsweise von 0,7g. Damit besitzt das Gehäuse 6 eine kaum zu beschleunigende Masse.

## Patentansprüche

1. Drahtloses Schrittmachernetzwerk zur Implantation in einen Körper eines Lebewesens und zur Steuerung einer Körperfunktion, wobei das Schrittmachernetzwerk aufweist:
eine elektronische Schrittmachereinheit (1), die als ein Master in dem Schrittmachernetzwerk agiert, beinhaltend
• einen Elektrodenabschnitt (2), der bestimmungsgemäß an einem ersten Körperabschnitt zu befestigen/anzuordnen ist, und
• eine Elektronik, die eingerichtet ist, eine Funktion des ersten Körperabschnittes, bevorzugt über den Elektrodenabschnitt, zu überwachen und/oder einen Impuls, insbesondere einen Spannungsimpuls, zu erzeugen und diesen über den Elektrodenabschnitt an den ersten Körperabschnitt abzugeben; und
eine elektronische Schrittmachereinheit (1'), die als ein Slave in dem Schrittmachernetzwerk agiert, beinhaltend
• einen Elektrodenabschnitt (2'), der bestimmungsgemäß an einem zweiten Körperabschnitt zu befestigen/anzuordnen ist, und
• eine mit dem Elektrodenabschnitt verbundene Elektronik, die eingerichtet ist, einen Impuls, insbesondere einen Spannungsimpuls, zu erzeugen und diesen über den Elektrodenabschnitt an den zweiten Körperabschnitt abzugeben; wobei
die als Master agierende Schrittmachereinheit (1) und die als Slave agierende Schrittmachereinheit (1') eingerichtet sind, zur Steuerung der Körperfunktion drahtlos zusammenzuwirken, indem die als Slave agierende Schrittmachereinheit
(i) Information über die Funktion des ersten Körperabschnittes von der als Master agierenden Schrittmachereinheit (1) und/oder über die Abgabe des Impulses an den ersten Körperabschnitt erhält, und
(ii) basierend auf der Information entscheidet, ob und/oder wann die Abgabe des Impuls an den zweiten Körperabschnitt erfolgt;
wobei die als Master agierende Schrittmachereinheit (1) und/oder die als Slave agierende Schrittmachereinheit (1') aufweist:
einen Energiespeicher zur Versorgung der entsprechenden Elektronik mit elektrischer Energie, der nach Entladung mit elektrischer Energie wieder aufgeladen werden kann; und
einen mit dem Energiespeicher elektrisch verbundenen Ladeimpulserzeugungsabschnitt, der derart eingerichtet ist, dass er an den Energiespeicher einen Ladeimpuls zum Wiederaufladen des Energiespeichers abgeben kann;
wobei
der Ladeimpulserzeugungsabschnitt einen Magnetisierungsabschnitt mit ausgerichteten magnetischen Domänen beinhaltet, der durch ein sich veränderndes Magnetfeld derart kontaktlos beeinflussbar ist, dass in ihm ab Erreichen einer bestimmten Feldstärke eine zur Erzeugung des Ladeimpulses führende über den Magnetisierungsabschnitt laufende Ummagnetisierungswelle, hervorgerufen durch die fortlaufend umgepolten magnetischen Domänen, auftritt.

2. Schrittmachernetzwerk nach Patentanspruch 1, wobei
die als Master agierende Schrittmachereinheit (1) eine Sendeeinheit aufweist und eingerichtet ist, die Information über die Sendeeinheit zu versenden; und
die als Slave agierende Schrittmachereinheit (1') eine Empfangseinheit aufweist und eingerichtet ist, die Information dadurch zu erhalten, dass sie die von der Sendeeinheit versendete Information über die Empfangseinheit empfängt.

3. Schrittmachernetzwerk nach Patentanspruch 1, wobei
die als Slave agierende Schrittmachereinheit (1') eine Detektionseinheit aufweist und eingerichtet ist, die Information dadurch zu erhalten, dass sie über die Detektionseinheit den von der als Master agierenden Schrittmachereinheit (1) abgegebenen Impuls detektiert.

4. Schrittmachernetzwerk gemäß einem der Patentansprüche 1 bis 4, wobei der Ladeimpulserzeugungsabschnitt mindestens eine Spule aufweist, die zu dem Magnetisierungsabschnitt derart räumlich, bevorzugt um den Magnetisierungsabschnitt diesen axial umschließend gewickelt, angeordnet ist, dass sie bei Auftreten der Ummagnetisierungswelle einen zu dem Ladeimpuls führenden Spannungsimpuls erzeugt.

5. Schrittmachernetzwerk gemäß Patentanspruch 4, wobei der Magnetisierungsabschnitt durch mechanische Bearbeitung derart ausgebildet ist, dass die magnetischen Domänen des Magnetisierungsabschnittes gleich ausgerichtet sind.

6. Schrittmachernetzwerk gemäß Patentanspruch 5, wobei der Magnetisierungsabschnitt einen hartmagnetischen Schalenbereich aufweist, der einen weichmagnetischen Kernbereich umschließt.

7. Schrittmachernetzwerk gemäß einem der Patentansprüche 1 bis 5, wobei der Magnetisierungsabschnitt mindestens ein Impulsdraht oder ein Wieganddraht ist.

8. Schrittmachernetzwerk gemäß Patentanspruch 7, wobei der Magnetisierungsabschnitt eine Vielzahl von Impulsdrähten oder eine Vielzahl von Wieganddrähten oder eine Kombination aus mindestens einem Impulsdraht und einem Wieganddraht aufweist.

9. Schrittmachernetzwerk gemäß Patentanspruch 8, wobei die Spule um die Vielzahl oder die Kombination von Drähten gewickelt ist, oder
mehrere Spulen vorgesehen sind, die jeweils um mindestens einen der Drähte gewickelt sind.

10. Schrittmachernetzwerk gemäß einem der Patentansprüche 1 bis 9, wobei der Ladeimpulserzeugungsabschnitt in einer Richtung, in der die mindestens eine Spule gewickelt ist, an mindestens einem Endabschnitt des Magnetisierungsabschnittes eine magnetische Sammellinse zur Bündelung und Führung des sich verändernden Magnetfeldes auf den Magnetisierungsabschnitt aufweist.

11. Schrittmachernetzwerk gemäß einem der Patentansprüche 1 bis 10, wobei die Elektronik der als Master agierende Schrittmachereinheit (1) und/oder der als Slave agierenden Schrittmachereinheit (1') zusammen mit dem jeweiligen Energiespeicher und dem jeweiligen Ladeimpulserzeugungsabschnitt von einer Hülle bzw. einem Gehäuse vollständig umgeben ist, die aus einem Material gebildet ist, das von dem Körper des Lebewesens nicht abgestoßen wird.

12. Schrittmachernetzwerk gemäß Patentanspruch 11, wobei das Material ein bevorzugt nicht-ferromagnetisches Metall, insbesondere Titan, oder eine, insbesondere Titan umfassende, Metalllegierung ist.

13. Schrittmachernetzwerk gemäß einem der vorhergehenden Ansprüche 1 bis 12, wobei die als Master agierende Schrittmachereinheit (1) und/oder die als Slave agierende Schrittmachereinheit (1')so ausgestaltet sind, dass die jeweilige Elektronik und/oder der jeweilige Ladeimpulserzeugungsabschnitt bis auf den Magnetisierungsabschnitt und, wenn bevorzugt vorgesehen, die mindestens eine magnetische Sammellinse keine Elemente aus ferromagnetischen Materialen aufweist.

14. Schrittmachernetzwerk gemäß einem der vorhergehenden Ansprüche 1 bis 13, wobei die Elektronik der als Master agierenden Schrittmachereinheit (1) und/oder der als Slave agierenden Schrittmachereinheit (1') eingerichtet ist, ein Signal auszusenden, welches die Qualität des Ladeimpulses anzeigt.

15. Schrittmachernetzwerk gemäß einem der Patentansprüche 1 bis 14, wobei der Energiespeicher der als Master agierenden Schrittmachereinheit (1) und/oder der als Slave agierenden Schrittmachereinheit (1') ein Akkumulator, beispielsweise ein Lithium-Ionen-Akkumulator, ist.

16. Schrittmachernetzwerk gemäß Patentanspruch 1 bis 14, wobei der Energiespeicher der als Master agierenden Schrittmachereinheit (1) und/oder der als Slave agierenden Schrittmachereinheit (1') ein Kondensator mit geringer Selbstentladung ist.

17. Schrittmachernetzwerk gemäß einem der Patentansprüche 10 bis 16, wobei die mindestens eine magnetische Sammellinse aus einem ferromagnetischen Metall gebildet ist, welches das Magnetfeld für den Magnetisierungsabschnitt bündelt.

18. Ladegerät für ein Schrittmachernetzwerk, wobei
das Ladegerät eingerichtet ist, ein sich mit einer Umpolfrequenz und bevorzugt Amplitude veränderndes Magnetfeld zu erzeugen, und
das Ladegerät bei bestimmungsgemäßer Verwendung auf einer Körperoberfläche eines Lebewesens oder in der Nähe der Körperoberfläche des Lebewesens derart angeordnet wird, dass das Magnetfeld in den Körper und die als Master agierende Schrittmachereinheit und/oder die als Slave agierende Schrittmachereinheit eines Schrittmachernetzwerks gemäß einem der Patentansprüche 1 bis 17 zur Beeinflussung des entsprechenden Ladeimpulserzeugungsabschnittes eindringt.

19. Ladegerät gemäß Patentanspruch 11, wobei die Umpolfrequenz in einem Bereich liegt von
• X bis 10kHz, wobei X>0 und X>= 0,1kHz, 0,2kHz, 0,3kHz,...,4,9kHz,..., oder 9,9kHz ist.

20. Ladegerät gemäß Patentanspruch 18 oder 19, wobei mehrere Spulen zur Erzeugung des sich verändernden Magnetfeldes vorgesehen sind, welche auf Basis des/der die Qualität des Ladeimpulses/der Ladeimpulse anzeigenden Signale für eine Optimierung des Ladeimpulses/der Ladeimpulse entsprechend angesteuert werden können.
